# EUROPÄISCHE PATENTANMELDUNG

(11) **EP 0 752 419 A2**
(43) Veröffentlichungstag der Anmeldung: **08.01.1997**
(21) Anmeldenummer: 96110203.5
(22) Anmeldetag: 25.06.1996
(51) Int. Cl.: C07D 207/24

(54) **Verfahren zur Herstellung von N-substituierten 4-Ketoprolin-Derivaten**

(30) Priorität: 04.07.1995 DE 19524339
(71) Anmelder: Degussa Aktiengesellschaft, 60311 Frankfurt (DE)
(72) Erfinder: Drauz, Karlheinz, Prof. Dr., 63579 Freigericht (DE); Stingl, Klaus, Dr., 63755 Alzenau (DE); Kottenhahn, Matthias, Dr., 63579 Freigericht (DE)

(57) **Zusammenfassung**

Verfahren zur Herstellung von N-geschützten 4-Ketoprolin-Derivaten der Formel I 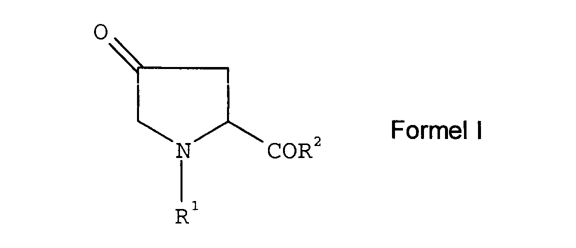 durch Oxidation von N-geschützten 4-Hydroxyprolin-Derivaten der Formel III 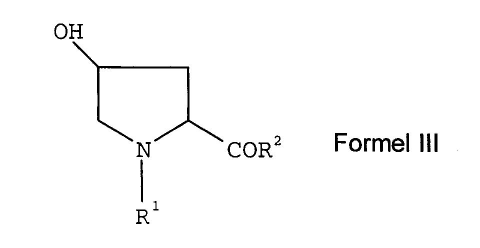 mit dem System TEMPO (2,2,6,6-Tetramethylpiperidin-1-oxyl)/NaOCl.

## Beschreibung

Die Erfindung betrifft ein neues Verfahren zur Herstellung von N-geschützten 4-Ketoprolin-Derivaten der allgemeinen Formel I, 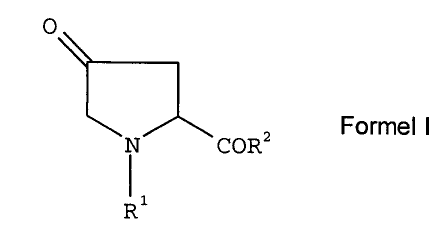 worin
- R¹ =: (C₁-C₆) Alkyl, CO-R³ oder Fluorenylmethoxycarbonyl,
- R² =: NH₂, OR⁴,
- R³ =: (H), (C₁-C₆ ) Alkyl, Phenyl, Benzyl, Benzyloxy, NH₂, NO₂-Phenyloxy, NO₂-Benzyloxy, (C₁-C₆) Alkoxy oder Phenyloxy,
- R⁴ =: (C₁-C₆) Alkyl, Benzyl, Phenyl, NO₂-Benzyl, NO₂-Phenyl oder Alkyl
bedeuten, bei dem man die Verbindung der Formel I aus den korrespondierenden N-geschützten 4-Hydroxyprolin-Derivaten durch Oxidation mit 2,2,6,6-Tetramethylpiperidin-1-oxyl in Gegenwart von Hypochlorit-Lösung erhält.

N-geschützte 4-Ketoprolin-Derivate der allgemeinen Formel I sind u. a. wichtige Ausgangsverbindungen zur Darstellung des ACE(Angiotensin Converting Enzyme)-Inhibitors Spirapril [7-(N-(1-(S)-Carboethoxy-3-phenylpropyl)-(S)-alanyl-1,4-dithia-7-azaspiro(4,4)nonan-8-(S)-carbonsäure] der Formel II, der zur Behandlung des Bluthochdruckes und von cardiovasculären Störungen dient (US-A-4,470,972).

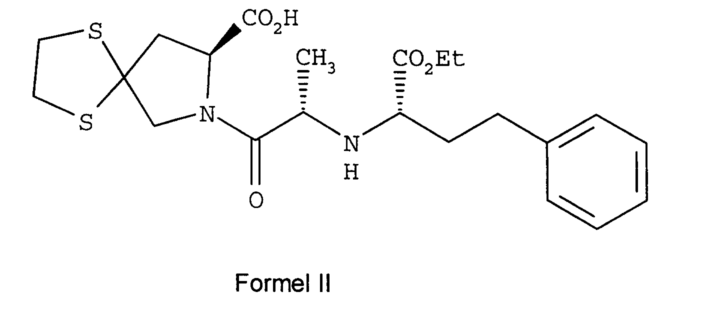

Verfahren zur Synthese von 4-Ketoprolin-Derivaten der allgemeinen Formel I basieren in der Regel auf schwermetallhaltigen Oxidationsmitteln, wie zum Beispiel diverse chromhaltige Oxidationssysteme (siehe US-A-4,296,113) oder RuO₂/NaOCl (siehe DD-A5 283 626). Diese Verfahren haben den Nachteil, daß sie zusätzlicher Sicherheitsmaßnahmen während der Reaktionsdurchführung bedürfen sowie einer aufwendigen und kostspieligen Schwermetallentsorgung nach Beendigung der Reaktion.

In DD-A5 283 626 ist ein schwermetallfreies Verfahren zur Herstellung von (2S)-N-Benzyloxycarbonyl-4-ketoprolin beschrieben, das als Oxidationsmittel einen Schwefeltrioxid-Pyridin-Komplex einsetzt. Dieses Verfahren hat den Nachteil, daß mit Pyridin ebenfalls eine stark umweltbelastende und für Menschen hochgiftige Substanz Verwendung findet.

Angesichts des diskutierten Standes der Technik bestand die Aufgabe der Erfindung darin, ein umweltschonendes Verfahren zur Herstellung von N-geschützten 4-Ketoprolin-Derivaten der allgemeinen Formel I anzugeben, das auf Schwermetalle und auf giftige Reagenzien wie Pyridin verzichtet, hohe Produktausbeuten und hohe Reinheiten der Produkte erzielt sowie ökonomische Vorteile aufweist.

Gelöst werden diese sowie weitere nicht einzeln genannte Aufgaben durch ein Verfahren mit den Merkmalen des kennzeichnenden Teils des Anspruchs 1. Vorteilhafte Verfahrensmodifikationen sind Gegenstand der auf Anspruch 1 rückbezogenen abhängigen Verfahrensansprüche.

Ferner sind im Verfahren der Erfindung als Produkt erhältliche neue Stoffe Gegenstand von Anspruch 8.

Es wurde nunmehr gefunden, daß man wesentlich umweltschonender und ökonomischer bei gleichzeitig hohen Produktausbeuten und hoher Reinheit der Produkte zu den Verbindungen der allgemeinen Formel I 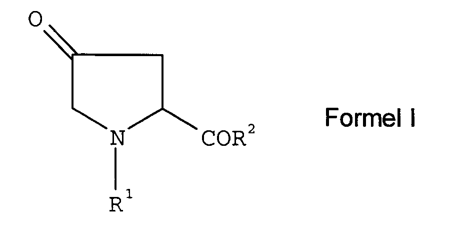 gelangen kann, wenn man deren korrespondierenden 4-Hydroxysubstituierten Analoga der allgemeinen Formel III, 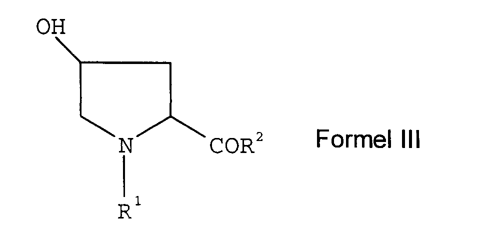 wobei R¹ und R² die oben angegebene Bedeutung haben,
durch Oxidation mit Hilfe von TEMPO (2,2,6,6-Tetramethylpiperidin-1-oxyl) in Gegenwart einer Hypochlorit-Lösung, wie beispielsweise NaOCl oder NaOBr (aus NaOCl und KBr generiert), in die N-geschützten 4-Ketoprolin-Derivate überführt.

Bei der Umsetzung werden von dem Nitroxyradikal (TEMPO) Mengen im Bereich von 0.05 - 10 mol%, vorteilhaft 0.5 - 2 mol%, von der Hypochloridlösung [NaOCl oder NaOBr (aus NaOCl und KBr generiert), normalerweise 12 - 13 %ig] 1 - 10 Äquivalente, vorteilhaft 1 - 4 Äquivalente, verwendet.

So werden im erfindungsgemäßen Verfahren beispielsweise hypochlorige-Säure oder hypobromige-Säure aus technischer Chlorbleichlauge oder technischer Chlorbleichlauge und KBr durch pH-Wert Herabsetzung mit Säure oder einer pH-Wert senkenden Base, vorteilhafterweise z. B. Natriumhydrogencarbonat, in situ generiert.

Die Oxidationsreaktion verläuft bei Temperaturen von -20 - 40 °C, besonders vorteilhaft zwischen 0 und 15 °C.

Als zusätzliche Lösungsmittel erwiesen sich grundsätzlich alle inerten organischen Lösungsmittel als geeignet. Besonders bevorzugt sind dabei organische Lösungsmittel, die mit der wässrigen Lösung ein Zweiphasensystem bilden. Die Reaktion kann aber auch in einem einphasigen Lösungsmittelgemisch (Wasser/org. Lösungsmittel) durchgeführt werden. Insbesondere jedoch sind halogenierte Kohlenwasserstoffe, wie zum Beispiel Dichlormethan, Chloroform, 1,1,1-Trichlorethan oder auch Ester der Ameisensäure und Essigsäure von Vorteil. Zur Vernichtung von überschüssiger hypochloriger Säure wird mit einem Reduktionsmittel, wie z. B. Natriumthiosulfatlösung aufgearbeitet. Nach Beendigung der Reaktion wird zur Abtrennung der organischen Phase ggf. mit Hilfe einer Base, wie beispielsweise einer anorganischen Base, wie NaOH, NaHCO₃, Na₂CO₃, KOH, oder einer organischen Phase, wie NEt₃ oder N-Methylmorpholin, auf ein pH-Bereich von 8 - 12, vorteilhafterweise auf pH = 9 - 10 gestellt. Anschließend stellt man den pH mit z. B. einer anorganischen Säure, wie HCl oder H₂SO₄ sauer und extrahiert mit einem produktlösenden organischen Lösungsmittel, wie beispielsweise Toluol, Methylenchlorid, Methylisobutylketon oder tert.Butylmethylether. Nach Entfernung des Lösungsmittels fallen die Oxidationsprodukte der allgemeinen Formel I in Form von Ölen bzw. Feststoffen in hoher Reinheit an.

Das erfindungsgemäße Verfahren wird im folgenden nochmals schematisch dargestellt: 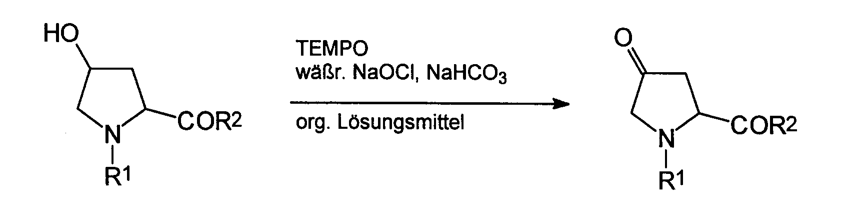 TEMPO = 2,2,6,6-Tetramethylpiperidin-1-oxyl

Die N-geschützten Hydroxyprolin-Derivate sind literaturbekannt und analog J. Med. Chem. 21, 607 (1978) herstellbar.

Die beschriebene Methodik soll an den nachfolgenden Beispielen verdeutlicht werden, ohne auf diese beschränkt zu sein.

### Ausführungsbeispiele

### Allgemeine Oxidationsvorschrift mit dem System TEMPO/NaOCl:

Zu einer 10 - 20 gew.-%igen Lösung von N-geschütztem Hydroxyprolinmethylester (1 Äquivalent) in Dichlormethan und 1 mol% TEMPO tropft man bei +5 °C eine mit Natriumhydrogencarbonat auf pH = 9.0 eingestellte techn. NaOCl-Lösung (2 Äquivalente) so zu, daß +15 °C nicht überschritten werden. Nach beendeter Zugabe wird noch 10 min weiter gerührt und anschließend werden die Phasen getrennt. Die wäßrige Phase wird noch einmal mit geringen Mengen Dichlormethan extrahiert und die vereinigten organischen Auszüge jeweils einmal mit 5 %iger wäßriger Natriumthiosulfat-Lösung und 5 %iger wäßriger Natriumhydrogencarbonat-Lösung gewaschen. Nach Trocknung des organischen Auszugs mit MgSO₄ und Entfernung des Lösungsmittels resultiert ein gelbes Öl oder Feststoff als Produkt.

Die nun aufgeführten Beispiele wurden analog der Allgemeinen Arbeitsvorschrift synthetisiert.

### Beispiel 1:

### (2S)-N-Boc-4-oxo-prolinmethylester

Aus 6.1 g (2S,4R)-N-Boc-4-hydroxyprolinmethylester erhält man 5.9 g (89 %) Produkt als hellgelbes Öl.
Reinheit: >95 % nach ¹H-NMR.

### Beispiel 2:

### (2S)-N-Benzoyl-4-oxo-prolinmethylester

Aus 6.23 g (2S,4R)-N-Benzoyl-4-hydroxyprolinmethylester erhält man 5.72 g (93 %) Produkt als hellbraunes Öl.
Reinheit: ∼93 % nach ¹H-NMR.

### Beispiel 3:

### (2S)-N-Benzyloxycarbonyl-4-oxo-prolinmethylester

Aus 29.0 g (2S,4R)-N-Benzyloxycarbonyl-4-hydroxyprolinethylester erhält man 25.3 g (88 %) Produkt als hellgelbes Öl.
Reinheit: 96 % nach HPLC und ¹H-NMR.

### Beispiel 4:

### (2S)-N-Benzyloxycarbonyl-4-oxo-prolinethylester

Aus 14.6 g (2S,4R)-N-Benzyloxycarbonyl-4-hydroxyprolinmethylester erhält man 12.5 g (86 %) Produkt als gelbes Öl.
Reinheit: 97 % nach HPLC.

### Beispiel 5:

### (2S)-N-Benzyloxycarbonyl-4-oxo-prolin-isopropylester

Aus 10.7 g (2S,4R)-N-Benzyloxycarbonyl-4-hydroxyprolinisopropylester erhält man 9.5 g (89 %) Produkt als hellbraunes Öl.
Reinheit: 92 % nach HPLC.

### Beispiel 6:

### (2S)-N-Benzyloxycarbonyl-4-oxo-prolin-n-butylester

Aus 16.1 g (2S,4R)-N-Benzyloxycarbonyl-4-hydroxyprolin-n-butylester erhält man 13.4 g (84 %) Produkt als hellbraunes Öl.
Reinheit: 96 % nach HPLC.

### Beispiel 7:

### (2S)-N-Benzyloxycarbonyl-4-oxo-prolinbenzylester

Aus 35.3 g (2S,4R)-N-Benzyloxycarbonyl-4-hydroxyprolinbenzylester erhält man 19.0 g (54 %) Produkt als farblosen Feststoff mit einem Schmelzpunkt von 56 °C.
Reinheit: >95 % nach ¹H-NMR.

### Beispiel 8:

### (2S)-N-Benzoyl-4-oxo-prolinbenzylester

Aus 16.2 g (2S,4R)-N-Benzoyl-4-hydroxyprolinbenzylester erhält man 13.2 g (82 %) Produkt als hellbraunes Öl.
Reinheit: 91 % nach HPLC.

## Patentansprüche

1. Verfahren zur Herstellung von N-substituierten 4-Ketoprolin-Derivaten der allgemeinen Formel I 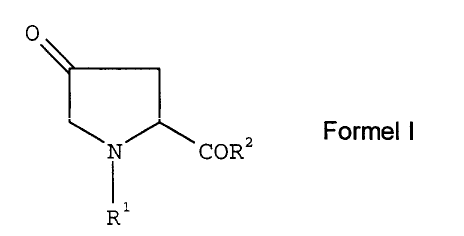 durch Oxidation der entsprechenden N-substituierten 4-Hydroxyprolin-Derivate der allgemeinen Formel III 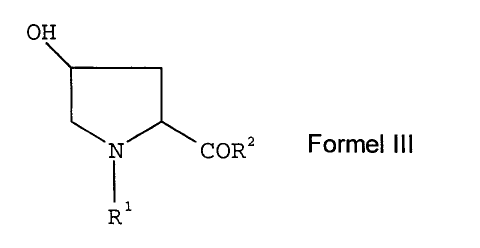 **dadurch gekennzeichnet,**
daß die Umsetzung in Gegenwart von 2,2,6,6-Tetramethylpiperidin-1-oxyl (TEMPO) und einer Hypochlorit-Lösung erfolgt.

2. Verfahren nach Anspruch 1,
**dadurch gekennzeichnet,**
daß Hypochlorit gleich NaOCl, NaOBr ist.

3. Verfahren nach Anspruch 1 oder 2,
**dadurch gekennzeichnet,**
daß man NaOBr aus NaOCl durch Zusatz von KBr generieren kann.

4. Verfahren nach Anspruch 1,
**dadurch gekennzeichnet,**
daß
R¹ = (C₁-C₆) Alkyl, CO-R³ oder Fluorenylmethoxycarbonyl,
R² = NH₂, OR⁴,
R³ = (H), (C₁-C₆ ) Alkyl, Phenyl, Benzyl, Benzyloxy, NH₂, NO₂-Phenyloxy, NO₂-Benzyloxy, (C₁-C₆) Alkoxy oder Phenyloxy,
R⁴ = (C₁-C₆) Alkyl, Benzyl, Phenyl, NO₂-Benzyl, NO₂-Phenyl oder Alkyl
sein kann.

5. Verfahren nach einem der vorausgegangenen Ansprüche,
**dadurch gekennzeichnet,**
daß die Reaktion in einem Zweiphasensystem bestehend aus einer wäßrigen Phase und einer mit dieser nicht mischbaren organischen Phase besteht.

6. Verfahren nach Anspruch 7,
**dadurch gekennzeichnet,**
daß man für die organische Phase ein halogeniertes Lösungsmittel, wie z. B. Dichlormethan, Chloroform, 1,1,1-Trichlorethan oder einen Ester der Ameisensäure oder der Essigsäure als Lösungsmittel verwendet.

7. Verfahren nach Anspruch 1 - 5,
**dadurch gekennzeichnet,**
daß die Reaktion bei Temperaturen von -20 °C bis 40 °C, bevorzugt bei 0 bis 15 °C für die Oxidation durchgeführt wird.

8. Nach einem der Ansprüche 1 - 8 beschriebenen Verfahren als Produkt erhältliche neue Verbindungen:
(2S)-N-Benzyloxycarbonyl-4-oxo-prolinisopropylester
(2S)-N-Benzyloxycarbonyl-4-oxo-prolin-n-butylester.
